# EUROPÄISCHE PATENTSCHRIFT

(11) **EP 1 275 390 B1**
(45) Veröffentlichungstag und Bekanntmachung des Hinweises auf die Patenterteilung: **02.11.2005**
(21) Anmeldenummer: 02021349.2
(22) Anmeldetag: 24.11.1994
(51) Int. Cl.: A61K 31/336, A61P 9/10

(54) **Arzneimittel zur Behandlung der Herzinsuffizienz**
Medicament for treatment of heart failure
Médicament pour le traitement de l'insuffisance cardiaque

(30) Priorität: 01.12.1993 DE 4340879
(43) Veröffentlichungstag der Anmeldung: 15.01.2003
(62) Teilanmeldung aus: 95901328.5
(73) Patentinhaber: Wolf, Horst P.O., 78476 Allensbach (DE)
(72) Erfinder: Wolf, Horst P.O., 78476 Allensbach (DE)
(74) Vertreter: Benz, Jürgen

(56) Entgegenhaltungen:
- WO-A-87/00751
- GARY D. LOPASCHUK ET AL.: "Response of isolated working hearts to fatty acids and carnitine palmitoyltransferase I inhibition during reduction of coronary flow in acutely and chronically diabetic rats" CIRCULATION RES., Bd. 65, Nr. 2, Seiten 378-387, XP009001162
- GARY D. LOPASCHUK ET AL.: "Glucose and palmitate oxidation in isolated working rat hearts reperfused after a period of transient global ischemia" CIRCULATION RES., Bd. 66, Nr. 2, Seiten 546-553, XP009001163
- WALL ET ALL: "Glucose oxidation rates in fatty acid-perfused isolated working hearts from diabetic rats" BIOCHIMICA ET BIOPHYSICA ACTA, Bd. 1006, 1989, Seiten 97-103, XP009001164
- REINAUER ET AL: "Influence of Carnitine Acyltransferase Inhibitors on the Performance and Metabolism of Rat Cardiac Muscle" J. CLIN.CHEM.CLIN.BIOCHEM., Bd. 28, 1990, Seiten 335-339, XP009001147
- Medline Abstract Accession No. 94002947 & Cardioscience, Band 4, Nr. 3, September 1993, Seiten 133-142 XP009001412
- H.RUPP ET AL.: "Metabolically-modulated growth and phenotype of the rat heart" EUROPEAN HEART JOURNAL, Bd. 13, Nr. supD, Seiten 56-61, XP009001422
- ARUN G. TAHILIANI ET AL.: "Diabetes-induced abnormalities in the myocardium" LIFE SCI., Bd. 38, Nr. 11, Seiten 959-974, XP009001413

## Beschreibung

Die Erfindung betrifft eine neue Verwendung von bekannten Oxirancarbonsäuren zur Herstellung von Arzneimitteln zur Behandlung der idiopathischen dilatativen Kardiomyopathie.

### Stand der Technik

In der EP-A-0 046 590 werden hypoglycämisch und hypoketonämisch und in der EP-A-0 231 367 hypocholesterolämisch und hypotriglyceridämisch wirksame phen(alk)oxy-substituierte Oxirancarbonsäuren beschrieben, die zur Behandlung des Diabetes Mellitus sowie solcher Erkrankungen eingesetzt werden sollen, die auf einer erhöhten Cholesterol- und/oder Triglycerid-Konzentration im Blut beruhen, wie Koronarsklerose und Arteriosklerose sowie alle damit zusammenhängenden krankhaften Veränderungen, die unter dem Sammelbegriff "koronare Herzkrankheiten" zusammengefaßt werden können. Die Herzleistung und Kontraktionskraft ist bei solchen Erkrankungen infolge der koronaren Mangeldurchblutung eingeschränkt, insbesondere bei diabetischen Herzen, Herzinfarkte sind häufig die Folge, wenn lokale Gefäßverschlüsse die Nährstoff-und Sauerstoffversorgung des Herzens unterbrechen.
Eine Herzmuskelerkrankung im Sinne einer primären Herzinsuffizienz, die auf einer Minderleistung der Herzmuskelzelle bei ungestörter Durchblutung beruht, liegt in diesen Fällen nicht vor.

Einige Publikationen in wissenschaftlichen Zeitschriften beschreiben eine andere Eigenschaft der bekannten Oxirancarbonsäuren, nämlich ihre Fähigkeit, durch Hemmung der Fettsäureoxidation die intracellulären Acylcarnitinkonzentrationen zu senken und über eine Veränderung des Gehaltes von Acylcarnitinen in den Muskelzellmembranen die elektrophysiologischen Prozesse bei der Herzkontraktion zu beeinflussen im Sinne einer "anti- arrhythmischen" Wirksamkeit (zusammenfassend in J.Clin.Invest.83, 1989, 927-936).

Einige neuere Arbeiten berichten auch über günstige Wirkungen der bekannten Oxirancarbonsäuren am ischämischen, hypoxischen und diabetischen Herzen (zusammenfassend G.D.Lopaschuk et al. in Current Concepts in Carnitine Research, 1992, 231-243) sowie über eine modifizerende Wirkung auf die Expression der verschiedenen Myosinisoenzyme im druckbelasteten Rattenherzen (H.Rupp et al., FASEB J. 6, 1992, 2349-2353, siehe auch N.S.Dhalla et al., Molecul.Cellul.Biochem. 116, 1992, 3-9).

### Beschreibung der Erfindung

Es wurde nun gefunden, daß die aus der EP-A-0 046590 bekannten Oxirancarbonsäuren beim Menschen mit dilatativer Kardiomyopathie (Herzinsuffizienz) die Funktion der kontraktilen Proteine so verändert, daß eine wesentliche Verbesserung der Herzleistung erreicht wird. Insbesondere verbesserte die Anwendung der bekannten Oxirancarbonsäuren die Herzleistungskurve bei herzinsuffizienten Patienten unter körperlicher Belastung (Belastungs-EKG).

Gegenstand der Erfindung ist daher die Verwendung von Oxirancarbonsäuren der Formel I 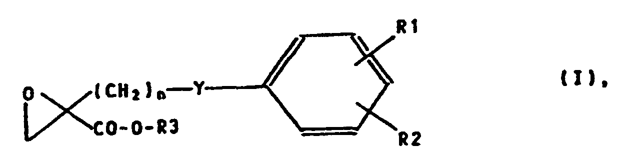 worin
- R1: ein Wasserstoffatom, ein Halogenatom, eine 1-4C-Alkylgruppe, eine 1-4C-Alkoxygruppe, eine Nitrogruppe oder eine Trifluormethylgruppe bedeutet,
- R2: eine der Bedeutungen von R1 hat,
- R3: ein Wasserstoffatom oder eine 1-4C-Alkylgruppe,
- Y: die Gruppierung -O-(CH₂)m-,
- m: 0 oder eine ganze Zahl von 1 bis 4 und
- n: eine ganze Zahl von 2 bis 8 bedeuten,
wobei die Summe von m und n eine ganze Zahl von 2 bis 8 ist,
und den pharmakologisch verträglichen Salzen der Carbonsäuren, zur Herstellung von Arzneimitteln für die Behandlung der idiopathischen dilatativen Kardiomyopathie.

Als 1-4C-Alkylgruppen kommen geradkettige oder verzweigte Alkylreste mit 1 bis 4 Kohlenstoffatomen in Betracht. Geradkettige Alkylreste sind beispielsweise der Methyl-, Ethyl-, n-Propyl- und n-Butylrest, von denen der Methyl- und der Ethylrest bevorzugt sind. Verzweigtkettige Alkylreste sind beispielsweise der Isopropyl-, Isobutyl-, sek.-Butyl- und tert.-Butylrest.

Als Alkylreste von 1-4C-Alkoxygruppen kommen sowohl geradkettige als auch verzweigtkettige Niederalkylgruppen in Frage. Die Methoxygruppe ist als 1-4C-Alkoxygruppe bevorzugt.

Halogenatome sind Fluor-, Chlor- und Bromatome, von denen Fluor, insbesondere Chlor bevorzugt sind.

Die Substituenten R1 und R2 des Phenylrings befinden sich bevorzugt in meta- oder para-Stellung zum (Alk)oxyalkylenoxirancarbonsäurerest.

Als Salze kommen Salze mit anorganischen und organischen Basen in Betracht. Als Kationen für die Salzbildung werden vor allem die Kationen der Alkalimetalle, Erdalkalimetalle oder Erdmetalle verwendet. Beispielsweise seien Salze von Lithium, Natrium, Kalium, Magnesium, Calcium und Aluminium genannt.

Bei der erfindungsgemäßen Verwendung der Verbindungen der Formel I zur Herstellung der vorstehend genannten Artneimittel werden die Verbindungen der Formel I (=Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt vorteilhafterweise zwischen 10 und 90 % beträgt.
Als besonders vorteilhafte galenische Formulierung ist die Weichgelatinekapsel anzusehen. In einer besonderer Ausführungsform enthält die Kapsel eine Lösung von Wirkstoff in Miglyol 812 mit einem Wirkstoffgehalt von 20,2 %.

Welche Hilfs- bzw. Trägerstoffe für die gewünschten Arzneimittelformulierungen geeignet sind, ist dem Fachmann aufgrund seines Fachwissens geläufig. Neben Lösungsmitteln. Gelbildnern, Suppositoriengrundlagen, Tabletten-Hilfstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, oder parenteral appliziert werden.

Im allgemeinen hat es sich in der Humanmedizin als vorteilhaft erwiesen, den oder die Wirkstoffe bei oraler Gabe in einer Tagesdosis von etwa 0,1 bis etwa 30, vorzugsweise 0,3 bis 15, insbesondere 0,6 bis 3 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die Wirkstoffe zur Behandlung der oben genannten Krankheit eingesetzt werden, so können die durch die erfindungsgemäße Verwendung der Wirkstoffe erhaltenen Arzneimittel auch einen oder mehrere andere pharmakologisch aktive Verbindungen, insbesondere andere sogenannte Herztherapeutika wie z.B. Herzglykoside, Nitropräparate, Diuretika, ACE-Hemmer, Calcium-Antagonisten, Beta-blocker, Antihypertonika, Vasodilatatoren und Mineralstoffe enthalten.

Eine Ausgestaltung der Erfindung ist die Verwendung von Oxirancarbonsäuren der Formel I, worin R1 und R2 meta- oder para-ständig sind und R1 ein Wasserstoffatom, ein Chloratom, eine Methylgruppe, eine Methoxygruppe, eine Nitrogruppe oder eine Trifluormethylgruppe, R2 ein Wasserstoffatom oder ein Chloratom, R3 ein Wasserstoffatom oder eine 1-4C-Alkylgruppe, Y die Gruppierung -O-(CH₂)m-, m 0 oder 1 und n eine ganze Zahl von 3 bis 7 bedeuten, wobei die Summe von m und n eine ganze Zahl von 3 bis 7 ist, und den pharmakologisch verträglichen Salzen der Carbonsäuren, zur Herstellung von Arzneimitteln für die Behandlung der idiopathischen dilatativen Kardiomyopathie.

Eine weitere Ausgestaltung der Erfindung ist die Verwendung von Oxirancarbonsäuren der Formel I, worin R1 meta- oder para-ständig ist und R1 ein Wasserstoffatom, ein Chloratom oder eine Trifluormethylgruppe, R2 ein Wasserstoffatom, R3 ein Wasserstoffatom, eine Methylgruppe oder eine Ethylgruppe, Y die Gruppierung -O- , n eine ganze Zahl von 4 bis 6 bedeuten, und den pharmakologisch verträglichen Salzen der Carbonsäuren, zur Herstellung von Arzneimitteln für die Behandlung der idiopathischen dilatativen Kardiomyopathie.

Eine bevorzugte Ausgestaltung der Erfindung ist die Verwendung einer oder mehrerer Oxirancarbonsäuren ausgewählt aus der Gruppe bestehend aus
2-(4-(3-Chlorphenoxy)-butyl)-oxiran-2-carbonsäureethylester,
2-(4-(3-Trifluormethylphenoxy)-butyl)-oxiran-2-carbonsäureethylester,
2-(6-(4-Chlorphenoxy)-hexyl)-oxiran-2-carbonsäureethylester,
2-(5-(4-Chlorphenoxy)-pentyl)-oxiran-2-carbonsäureethylester,
2-(6-(3,4-Dichlorphenoxy)-hexyl-)-oxiran-2-carbonsäureethylester,
2-(6-(4-Fluorphenoxy)-hexyl)-oxiran-2-carbonsäureethylester und
2-(6-Phenoxyhexyl)-oxiran-2-carbonsäureethylester
sowie den entsprechenden Oxirancarbonsäuren und ihren pharmakologisch verträglichen Salzen.
Eine besonders bevorzugte Ausgestaltung der Erfindung ist die Verwendung des (+)-Enantlomers des 2-(6-(4-Chlorphenoxy)-hexyl)-oxiran-2-carbonsäureethylesters.

## Patentansprüche

1. Verwendung von Oxirancarbonsäuren der Formel I 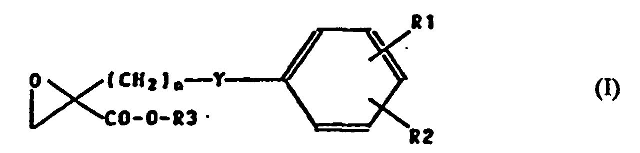 worin
R1 ein Wasserstoffatom, ein Halogenatom, eine 1-4C-Alkylgruppe, eine 1-4C-Alkoxygruppe, eine Nitrogruppe oder eine Trifluormethylgruppe bedeutet,
R2 eine der Bedeutungen von R1 hat,
R3 ein Wasserstoffatom oder eine 1-4C-Alkylgruppe,
Y eine Gruppierung -O-(CH₂)ₘ-,
m 0 oder eine ganze Zahl von 1 bis 4 und
n eine ganze Zahl von 2 bis 8 bedeuten,
wobei die Summe von m und n eine ganze Zahl von 2 bis 8 ist, und den pharmakologisch verträglichen Salzen der Carbonsäuren zur Herstellung eines Arzneimittels für die Behandlung der idiopathischen dilatativen Kardiomyopathie beim Menschen.

2. Verwendung der Oxirancarbonsäuren der Formel I gemäß Anspruch 1, worin R1 und R2 meta- oder para-ständig sind und R1 ein Wasserstoffatom, ein Chloratom, eine Methylgruppe, eine Methoxygruppe, eine Nitrogruppe oder eine Trifluormethylgruppe. R2 ein Wasserstoffatom oder ein Chloratom, R3 ein Wasserstoffatom oder eine 1-4C-Alkylgruppe, Y die Gruppierung -O-(CH₂)m-, m 0 oder 1 und n eine ganze Zahl von 3 bis 7 bedeuten, wobei die Summe von m und n eine ganze Zahl von 3 bis 7 ist, und den pharmakologisch verträglichen Salzen der Carbonsäuren.

3. Verwendung von Oxirancarbonsäuren der Formel I gemäß Anspruch 1, worin R1 meta- oder paraständig ist und R1 ein Wasserstoffatom, ein Chloratom oder eine Trifluormethylgruppe, R2 ein Wasserstoffatom, R3 ein Wasserstoffatom, eine Methylgruppe oder eine Ethylgruppe, Y die Gruppierung -O-, und n eine ganze Zahl von 4 bis 6 bedeuten, und den pharmazeutisch verträglichen Salzen der Carbonsäuren.

4. Verwendung einer oder mehrerer Verbindungen der Formel I gemäß Anspruch 1, ausgewählt aus der Gruppe bestehend aus
2-(4-(3-Chlorphenoxy)-butyl)-oxiran-2-carbonsäureethylester,
2-(4-(3-Trifluormethylphenoxy)-butyl)-oxiran-2-carbonsäureethylester,
2-(6-(4-Chlorphenoxy)-hexyl)-oxiran-2-carbonsäureethylester,
2-(5-(4-Chlorphenoxy)-pentyl)-oxiran-2-carbonsäureethylester,
2-(6-(3,4-Dichlorphenoxy)-hexyl)-oxiran-2-carbonsäureethylester,
2-(6-(4-Fluorphenoxy)-hexyl)-oxiran-2-carbonsäureethylester, und
2-(6-Phenoxyhexyl)-oxiran-2-carbonsäureethylester
sowie den entsprechenden Oxirancarbonsäuren und ihren pharmakologisch verträglichen Salzen.

5. Verwendung gemäß Anspruch 1 des (+)-Enantiomers des 2-(6-(4-Chlorphenoxy)-hexyl)-oxiran-2-carbonsäureethylesters.

## Claims

1. Use of oxiranecarboxylic acids of the formula I 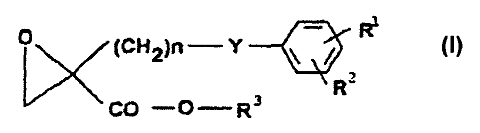 in which
R1 denotes a hydrogen atom, a halogen atom, a 1 - 4 C alkyl group, a 1 - 4 C alkoxy group, a nitro group or a trifluoromethyl group,
R2 has one of the meanings of R1,
R3 denotes a hydrogen atom or a 1 - 4 C alkyl group,
Y denotes an - O - (CH₂)ₘ - group,
m denotes 0 or an integer from 1 to 4 and
n denotes an integer from 2 to 8,
where the sum of m and n is an integer from 2 to 8,
and the pharmacologically tolerated salts of the carboxylic acids
for the preparation of a medicament for the treatment of idiopathic dilatative cardiomyopathy.

2. Use of the oxiranecarboxylic acids of the formula I according to Claim 1 in which
R1 and R2 are in the meta- or para-position and
R1 denotes a hydrogen atom, a chlorine atom, a methyl group, a methoxy group, a nitro group or a trifluoromethyl group,
R2 denotes a hydrogen atom or a chlorine atom,
R3 denotes a hydrogen atom or a 1 - 4 C alkyl group,
Y denotes the - O - (CH₂)ₘ - group,
m denotes 0 or 1 and n denotes an integer from 3 to 7, where the sum of m and n is an integer from 3 to 7,
and the pharmacologically tolerated salts of the carboxylic acids.

3. Use of the oxiranecarboxylic acids of the formula I according to Claim 1 in which
R1 is in the meta- or para-position, and
R1 denotes a hydrogen atom, a chlorine atom or a trifluoromethyl group,
R2 denotes a hydrogen atom,
R3 denotes a hydrogen atom, a methyl group or an ethyl group,
Y denotes the - O - group, and
n denotes an integer from 4 to 6,
and the pharmaceutically tolerated salts of the carboxylic acids.

4. Use of one or more compounds of the formula I according to Claim 1, selected from the group consisting of:
ethyl 2-(4-(3-chlorophenoxy)butyl)oxirane-2-carboxylate,
ethyl 2-(4-(3-trifluoromethylphenoxy)butyl)oxirane-2-carboxylate,
ethyl 2-(6-(4-chlorophenoxy)hexyl)oxirane-2-carboxylate,
ethyl 2-(5-(4-chlorophenoxy)pentyl)oxirane-2-carboxylate,
ethyl 2-(6-(3,4-dichlorophenoxy)hexyl)oxirane-2-carboxylate,
ethyl 2-(6-(4-fluorophenoxy)hexyl)oxirane-2-carboxylate, and
ethyl 2-(6-phenoxyhexyl)oxirane-2-carboxylate
and the corresponding oxiranecarboxylic acids and pharmacologically tolerated salts thereof.

5. Use according to Claim 1 of the (+) enantiomer of ethyl 2-(6-(4-chlorophenoxy)hexyl)-oxirane-2-carboxylate.

## Revendications

1. Utilisation d'acides oxiranecarboxyliques de la formule 1 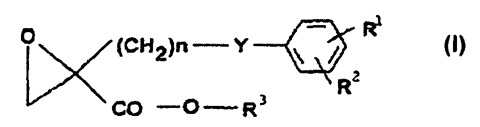 dans laquelle
R1 représente un atome d'hydrogène, un atome d'halogène, un groupe alkyle comportant 1 - 4 atomes de C, un groupe alkoxy comportant 1 - 4 atomes de C, un groupe nitro ou un groupe trifluorométhyle,
R2 présente l'une des significations de R1,
R3 représente un atome d'hydrogène ou un groupe alkyle comportant 1 - 4 atomes de C,
Y représente un groupe - O - (CH₂)ₘ -,
m représente 0 ou un entier de 1 à 4 et
n représente un entier de 2 à 8,
où la somme de m et de n est un entier de 2 à 8,
et des sels tolérés pharmacologiquement des acides carboxyliques
pour la préparation d'un médicament pour le traitement d'une cardiomyopathie dilatative idiopathique.

2. Utilisation des acides oxiranecarboxyliques de la formule I selon la revendication 1 dans laquelle
R1 et R2 sont à la position méta- ou para- et
R1 représente un atome d'hydrogène, un atome de chlore, un groupe méthyle, un groupe méthoxy, un groupe nitro ou un groupe trifluorométhyle,
R2 représente un atome d'hydrogène ou un atome de chlore,
R3 représente un atome d'hydrogène ou un groupe alkyle comportant 1 - 4 atomes de C,
Y représente le groupe - O - (CH₂)ₘ -,
m représente 0 ou 1 et n représente un entier de 3 à 7, où la somme de m et de n est un entier de 3 à 7,
et des sels tolérés pharmacologiquement des acides carboxyliques.

3. Utilisation des acides oxiranecarboxyliques de la formule I selon la revendication 1 dans laquelle
R1 est à la position méta- ou para-, et
R1 représente un atome d'hydrogène, un atome de chlore ou un groupe trifluorométhyle,
R2 représente un atome d'hydrogène,
R3 représente un atome d'hydrogène, un groupe méthyle ou un groupe éthyle,
Y représente le groupe - O -, et
n représente un entier de 4 à 6,
et des sels tolérés pharmaceutiquement des acides carboxyliques.

4. Utilisation d'un ou de plusieurs composés de la formule I selon la revendication 1, choisis parmi le groupe comprenant:
2-(4-(3-chlorophénoxy)butyl)oxirane-2-carboxylate d'éthyle,
2-(4-(3-trifluorométhylphénoxy)butyl)oxirane-2-carboxylate d'éthyle,
2-(6-(4-chlorophénoxy)hexyl)oxirane-2-carboxylate d'éthyle,
2-(5-(4-chlorophénoxy)pentyl)oxirane-2-carboxylate d'éthyle,
2-(6-(3,4-dichlorophénoxy)hexyl)oxirane-2-carboxylate d'éthyle,
2-(6-(4-fluorophénoxy)hexyl)oxirane-2-carboxylate d'éthyle, et
2-(6-phénoxyhexyl)oxirane-2-carboxylate d'éthyle
et des acides oxiranecarboxyliques correspondants et de leurs sels tolérés pharmacologiquement.

5. Utilisation selon la revendication 1 du (+) énantiomère 2-(6-(4-chlorophénoxy)-hexyl)oxirane-2-carboxylate d'éthyle.
